# EUROPÄISCHE PATENTSCHRIFT

(11) **EP 4 590 227 B1**
(45) Veröffentlichungstag und Bekanntmachung des Hinweises auf die Patenterteilung: **11.11.2026**
(21) Anmeldenummer: 23836358.4
(22) Anmeldetag: 15.12.2023
(51) Int. Cl.: A61B 34/00, A61B 17/00

(54) **LENKGETRIEBE FÜR EIN CHIRURGISCHES INSTRUMENT**
STEERING MECHANISM FOR A SURGICAL INSTRUMENT
MÉCANISME DE DIRECTION POUR UN INSTRUMENT CHIRURGICAL

(30) Priorität: 20.12.2022 DE 102022134207
(43) Veröffentlichungstag der Anmeldung: 30.07.2025
(73) Patentinhaber: KARL STORZ SE & Co. KG, 78532 Tuttlingen (DE)
(72) Erfinder: GRÜNER, Sven, 78532 Tüttlingen (DE); STEFAN, Jochen, 78532 Tüttlingen (DE)
(86) Internationale Anmeldenummer: PCT/EP2023/086012
(87) Internationale Veröffentlichungsnummer: WO 2024/132906

(56) Entgegenhaltungen:
- CN-A- 103 552 088
- DE-A1- 102019 121 092
- DE-B3- 102021 119 527
- US-A1- 2012 239 060

## Beschreibung

Die vorliegende Erfindung betrifft ein Lenkgetriebe für ein chirurgisches Instrument. Ferner betrifft die vorliegende Erfindung ein chirurgisches Instrument zur Kopplung mit einem solchen Lenkgetriebe. Zudem betrifft die vorliegende Erfindung ein steriles Antriebssystem mit einem Lenkgetriebe und einem chirurgischen Instrument. Die vorliegende Erfindung betrifft auch ein Verfahren zur Montage eines solchen sterilen Antriebssystems.

Aus dem Stand der Technik sind chirurgische Instrumente bekannt, die manuell oder von einem Roboter geführt werden können und die Werkzeuge aufweisen, deren Werkzeugspitze mittels mehrerer ineinandergreifende Schwenkglieder verschwenkt werden kann. Diese Schwenkglieder sind mit einer Vielzahl von Lenkdrähte oder Lenkseilen verbunden, um eine feinfühlige Steuerung der Werkzeugspitze zu erreichen. Mit den Lenkdrähten kann eine gleichmäßige Kraftverteilung in allen Abwicklungsrichtungen erreicht werden.

CN 103552088 B beschreibt ein Lenkgetriebe für ein chirurgisches Instrument mit einer Taumelscheibe.

Aus US 7,699,855 B2 ist ein chirurgisches Instrument bekannt, das eine Schnittstelle aufweist, um das Instrument mit einem Roboterarm verbinden zu können. Sämtliche Antriebe für die Funktionen des Instruments sind an dem Roboterarm angeordnet. Die Übertragung der Drehwinkel von den Antrieben zum Instrument erfolgt über Kupplungsscheiben in einer gemeinsamen Trennebene. WO 2014/004242 A und US 2017/0165017 A1 offenbaren weitere Ausführungen von Schnittstellen zwischen einem chirurgischen Instrument und den roboterseitig angeordneten Antrieben zum Antreiben des chirurgischen Instruments.

Aus DE 102019 121 092 A1 ist ein chirurgisches Instrument bekannt, dass ein Lenkgetriebe aufweist. Mit dem Lenkgetriebe können die Stellwinkel von zwei Antrieben direkt auf eine räumlich verstellbare Scheibe (Taumelscheibe) übertragen werden, um diese zur Steuerung der Werkzeugspitze auszurichten. Dazu werden an der Taumelscheibe Lenkdrähte befestigt, sodass sich die Werkzeugspitze durch Ausrichtung der Taumelscheibe stufenlos und flüssig steuern lässt. Auch aus US 10,105,128 B ist ein Getriebe für ein chirurgisches Instrument bekannt. Das Getriebe dieses chirurgischen Instruments umfasst eine verlagerbare Scheibe und Gelenksstangen, die zum Bewegen der verlagerbaren Scheibe angeordnet sind.

Ausgehend von diesem Stand der Technik ist es Aufgabe der vorliegenden Erfindung, ein Lenkgetriebe mit einer Schnittstelle für ein chirurgisches Instrument bereitzustellen, über die eine instrumentenseitig angeordnete Taumelscheibe ansteuerbar ist.

Diese Aufgabe wird mit einem Lenkgetriebe für ein chirurgisches Instrument gemäß dem Patentanspruch 1 gelöst. Weitere Ausführungsformen sind in den abhängigen Ansprüchen angegeben.

Das erfindungsgemäße Lenkgetriebe weist ein Getriebegehäuse und eine Schnittstelle zur Kopplung mit einem chirurgischen Instrument auf. Die Schnittstelle ist derart ausgebildet, dass das chirurgische Instrument in einer Richtung senkrecht zur Längsachse des Lenkgetriebes mit dem Lenkgetriebe koppelbar ist. Die Schnittstelle umfasst einen Gehäusebereich des Getriebegehäuses und eine drehbar gelagerte Lenkgabel zur Betätigung einer Lenkeinrichtung des chirurgischen Instruments. Der Gehäusebereich des Getriebegehäuses ist zur Kopplung mit einem Gehäuse des chirurgischen Instruments ausgebildet. Die drehbar gelagerte Lenkgabel ist mit einer Taumelscheibe der Lenkeinrichtung des chirurgischen Instruments über die Schnittstelle koppelbar.

Das erfindungsgemäße Lenkgetriebe ist zur Ansteuerung einer Lenkeinrichtung eines chirurgischen Instruments über die Schnittstelle ausgebildet. Insbesondere kann über die Schnittstelle des Lenkgetriebes eine Taumelscheibe der Lenkeinrichtung des chirurgischen Instruments gesteuert werden. Das chirurgische Instrument kann von oben auf das Lenkgetriebe aufgesetzt und über die Schnittstelle mit dem Lenkgetriebe gekoppelt werden. Die drehbar gelagerte Lenkgabel ist nach oben geöffnet, so dass die Taumelscheibe von oben in die Lenkgabel eingesetzt werden kann. Aufgrund der Schnittstelle des Lenkgetriebes kann das chirurgische Instrument relativ einfach und damit kostengünstig aufgebaut werden. Das chirurgische Instrument kann demnach als sogenannte Limited-use-Artikel ausgeführt sein. Die Schnittstelle des Lenkgetriebes ermöglicht einen intraoperativen Wechsel des chirurgischen Instruments. Das sterile Lenkgetriebe bleibt während der Operation durchgängig mit einer roboterseitigen angeordneten Antriebseinheit gekoppelt, während verschiedene chirurgische Instrumente intraoperativ mit dem Lenkgetriebe gekoppelt werden können. Das erfindungsgemäße Lenkgetriebe ist zudem mehrfach sterilisierbar.

Die Lenkgabel kann derart ausgebildet sein, dass die Lenkgabel in einer Richtung senkrecht zur Längsachse mit der Taumelscheibe des chirurgischen Instruments in Eingriff bringbar ist. Das chirurgische Instrument kann dementsprechend in einer Richtung senkrecht zur Längsachse des Lenkgetriebes auf das Lenkgetriebe aufgesetzt werden. Dabei kann die drehbar gelagerte Lenkgabel mit der Taumelscheibe gekoppelt werden, sodass die Bewegungen der Lenkgabel auf die instrumentenseitig angeordnete Taumelscheibe übertragen werden können. Auch das Abnehmen des chirurgischen Instruments von dem Lenkgetriebe erfolgt in einer Richtung senkrecht zur Längsachse des Lenkgetriebes. Die Wechsel des chirurgischen Instruments können intraoperativ stattfinden, wobei das Lenkgetriebe während der Operation durchgängig an der Antriebseinheit verbleibt.

Die Lenkgabel kann eine Nut haben. Die Nut kann axiale Kontaktflächen für die Taumelscheibe aufweisen. Die axialen Kontaktflächen de Nut können einander zugewandt sein. Die axialen Kontaktflächen können sich parallel zueinander erstrecken. Die Nut kann an der Innenseite der Lenkgabel ausgebildet sein. Die Nut kann sich vollständig entlang der Innenseite der Lenkgabel erstrecken. Durch die axialen Kontaktflächen zur Anlage der Taumelscheibe kann ein Verkanten der Taumelscheibe in der Lenkgabel bei der Übertragung von Kipp- oder Rotationsbewegungen von der Lenkgabel auf die Taumelscheibe verhindert werden.

Die Schnittstelle kann eine Zugklaue aufweisen, die zur Kopplung mit der Lenkeinrichtung des chirurgischen Instruments ausgebildet ist. Die Zugklaue kann eine Aufnahme aufweisen, die derart ausgebildet ist, dass die Zugklaue in einer Richtung senkrecht zur Längsachse des Lenkgetriebes mit der Lenkeinrichtung des chirurgischen Instruments in Eingriff bringbar ist.

Das Lenkgetriebe kann wenigstens einen Haltebügel aufweisen, der um eine erste Drehachse drehbar an dem Getriebegehäuse gelagert ist. Die Lenkgabel kann um eine zweite Drehachse drehbar an dem Haltebügel gelagert sein. Der Haltebügel kann zwei Verbindungsabschnitte und einen sich zwischen den Verbindungsabschnitten erstreckenden Lagerabschnitt aufweisen. An dem Lagerabschnitt kann die Lenkgabel drehbar gelagert sein. Der Haltebügel kann somit um die erste Drehachse verschwenkt werden. Die zweite Drehachse der Lenkgabel kann die von den Lagerwellen definierte erste Drehachse schneiden. Auf diese Weise kann eine kardanische Lagerung der Lenkgabel erreicht werden.

Das Lenkgetriebe kann ein erstes Antriebsrad und ein zweites Antriebsrad aufweisen, die jeweils über eine Lagerwelle an dem Getriebegehäuse um die erste Drehachse drehbar gelagert sind. Der Haltebügel kann drehbar an den Lagerwellen der Antriebsräder gelagert sein. Der Haltebügel kann über seine Verbindungsabschnitte mit den Lagerwellen verbunden sein, so dass der Haltebügel über die Verbindungsabschnitte drehbar an den Lagerwellen gelagert ist.

Das erste Antriebsrad und das zweite Antriebsrad können jeweils eine Kegelverzahnung aufweisen. Die Kegelverzahnungen an dem ersten Antriebsrad und an dem zweiten Antriebsrad können jeweils nur segmentweise ausgebildet sein. Die Kegelverzahnungen können in Form eines Kreissegments ausgebildet sein. Diese Kreissegmente können sich beispielsweise über einen Winkelbetrag von 60° bis 120° erstrecken.

Die Lenkgabel kann wenigstens zwei Kegelverzahnungsabschnitte aufweisen. Auch die Kegelverzahnungsabschnitte können segmentweise ausgebildet sein. Dies bedeutet, dass die Kegelverzahnungsabschnitte in Form von Kreissegmenten ausgebildet sein können. Die Kegelverzahnungsabschnitte können sich beispielswiese über einen Winkelbetrag über 60° bis 120° erstrecken. Die Kegelverzahnungsabschnitte können mit den Kegelverzahnungen an den Antriebsrädern in Eingriff stehen, um die Lenkgabel bewegen zu können.

Durch die segmentweise ausgebildeten Kegelverzahnungen und Kegelverzahnungsabschnitte kann Bauraum eingespart werden. Das Lenkgetriebe kann ohne vollständige Kegelzahnräder ausgeführt werden, wodurch sich der benötigte Bauraum entsprechend reduziert. Darüber hinaus können durch die segmentweise Ausbildung der Kegelverzahnungen und der Kegelverzahnungsabschnitte Kollisionen dieser Elemente mit Lenkdrähten des chirurgischen Instruments vermieden werden, wenn das chirurgische Instrument über die Schnittstelle mit dem Lenkgetriebe gekoppelt ist.

Die Kegelverzahnungsabschnitte können einstückig mit der Lenkgabel ausgebildet sein. Die Kegelverzahnungsabschnitte können auch unabhängig von der Lenkgabel als separates Bauteil oder separate Bauteile ausgebildet sein. In diesem Fall können die Kegelverzahnungsabschnitte mit der Lenkgabel drehmomentübertragend verbunden werden. Mit einer zwei- oder mehrteiligen Ausführung der Lenkgabel und der damit zu verbindenden Kegelverzahnungsabschnitte kann die Herstellung dieser Bauteile einfach gehalten werden.

Das Lenkgetriebe kann ein erstes Zahnrad, ein zweites Zahnrad, eine erste Antriebsspindel und eine zweite Antriebsspindel aufweisen. Das erste Zahnrad kann der ersten Antriebsspindel und das zweite Zahnrad der zweiten Antriebsspindel zugeordnet sein. Das erste Zahnrad und das zweite Zahnrad können an den Lagerwellen der Antriebsräder drehbar gelagert sein. Die Zugklaue kann über eine dritte Antriebsspindel antreibbar sein. Die Zugklaue kann über die dritte Antriebsspindel entlang der Längsachse des Lenkgetriebes verlagert werden.

Die Antriebsspindeln können sich parallel zur Längsachse des Lenkgetriebes durch das Getriebegehäuse erstrecken und an ihren Enden jeweils eine Antriebsscheibe aufweisen, die mit Motortreibscheiben koppelbar sind.

Die Schnittstelle kann wenigstens ein Stirnrad zum Antreiben eines Schafts des chirurgischen Instruments aufweisen. Das Stirnrad kann von einer weiteren Antriebsscheibe antreibbar sein. Das Stirnrad kann über ein Getriebe mit der Antriebsscheibe gekoppelt sein. Dieses Getriebe kann von wenigstens zwei Zahnrädern gebildet werden. Das Stirnrad kann über eine Welle mit dem Getriebe verbunden sein. Mit dem Getriebe kann beispielsweise ein Versatz in einer Richtung senkrecht zur Längsachse des Lenkgetriebes zwischen dem Stirnrad und der Antriebsscheibe ausgeglichen werden.

Das Lenkgetriebe kann eine weitere Schnittstelle zur Verbindung mit einer Antriebseinheit aufweisen. Diese Schnittstelle kann die Antriebsscheiben zur Kopplung mit den Motortreibscheiben einer Antriebseinheit aufweisen. Das Lenkgetriebe kann somit eine erste Schnittstelle zur Kopplung mit dem chirurgischen Instrument und eine zweite Schnittstelle zur Kopplung mit der Antriebseinheit aufweisen. Diese Antriebseinheit kann roboterseitigen angeordnet sein. Die Kopplung mit der Antriebseinheit kann in Richtung der Längsachse des Lenkgetriebes erfolgen. Die Richtung der Längsachse kann der Richtung der Drehachsen der Antriebsspindeln des Lenkgetriebes entsprechen, die parallel zur Längsachse des Lenkgetriebes verlaufen.

Mit der zweiten Schnittstelle kann das Lenkgetriebe die unsterilen Antriebe an der Antriebseinheit abdecken, sodass das Lenkgetriebe während einer Operation durchgängig mit der Antriebseinheit bzw. den darin enthaltenen Antrieben gekoppelt bleiben kann. An der zweiten Schnittstelle des Lenkgetriebes können die Antriebsscheiben der Antriebsspindeln angeordnet sein. Insbesondere kann die Schnittstelle sich eine im wesentlichen senkrecht zur Längsachse des Lenkgetriebes verlaufende Fläche des Getriebegehäuses umfassen, an der die Antriebsscheiben angeordnet sind. An der Antriebseinheit können die Motortreibscheiben angeordnet sein, die drehmomentübertragend mit den Antriebsscheiben gekoppelt werden können, um die Antriebsspindeln des Lenkgetriebes antreiben zu können.

Die vorliegende Erfindung betrifft ferner ein chirurgisches Instrument zur Kopplung mit einer Schnittstelle eines Lenkgetriebes gemäß der voranstehend beschriebenen Art. Das chirurgische Instrument umfasst ein Gehäuse und eine in dem Gehäuse angeordnete Lenkeinrichtung. Die Lenkeinrichtung kann zumindest eine Schaftwelle, eine Taumelscheibe und mit der Taumelscheibe verbundene Lenkdrähte aufweisen. Das Gehäuse hat eine Öffnung, über die die Lenkeinrichtung mit dem Lenkgetriebe koppelbar ist.

Das chirurgische Instrument ist so ausgebildet, dass es in einer Richtung senkrecht zur Längsachse des chirurgischen Instruments mit der Schnittstelle des Lenkgetriebes koppelbar ist. Aufgrund des Lenkgetriebes bzw. der an dem Lenkgetriebe ausgebildeten Schnittstelle kann das chirurgische Instrument den voranstehend beschriebenen einfachen und damit kostengünstigen Aufbau aufweisen. Das chirurgische Instrument selbst kann aufgrund seines einfachen Aufbaus ein sogenannter Limited-use-Artikel sein.

Die Taumelscheibe kann einen umlaufenden Vorsprung aufweisen, der axiale Kontaktflächen für die Lenkgabel aufweist. Die axialen Kontaktflächen des umlaufenden Vorsprungs können voneinander wegweisen. Die axialen Kontaktflächen können zueinander entgegengesetzt an dem Vorsprung ausgebildet sind. Die axialen Kontaktflächen können sich parallel zueinander erstrecken. Der umlaufende Vorsprung der Taumelscheibe kann mit der Nut an der Innenseite der Lenkgabel in Eingriff gebracht werden. Durch die korrespondierenden axialen Kontaktflächen an der Nut der Lenkgabel und den axialen Kontaktflächen an dem umlaufenden Vorsprung der Taumelscheibe kann ein Verkanten bei der Übertragung von Kipp- und Rotationsbewegungen von der Lenkgabel auf die Taumelscheibe des chirurgischen Instruments verhindert werden.

Der umlaufende Vorsprung der Taumelscheibe kann Befestigungselemente zur Befestigung der Lenkdrähte an der Taumelscheibe aufweisen. Da die Befestigungselemente in dem umlaufenden Vorsprung der Taumelscheibe aufgenommen werden können, können die Befestigungselemente im mit der Taumelscheibe gekoppelten Zustand der Lenkgabel zusammen mit dem umlaufenden Vorsprung platzsparend in der Nut der Lenkgabel untergebracht werden. Dazu können in dem umlaufenden Vorsprung Öffnungen ausgebildet sein, die sich in radialer Richtung in die Taumelscheibe hinein erstrecken. Die Befestigungselemente können in den Öffnungen aufgenommen sein und eine Klemmkraft für die an der Taumelscheibe zu befestigenden Lenkdrähte bereitstellen. Die Befestigungselemente können Schrauben sein. Dementsprechend können die Öffnungen in der Taumelscheibe ein Innengewinde aufweisen. Insbesondere können die Befestigungselemente Madenschrauben sein.

Die Lenkeinrichtung kann wenigstens eine Zughülse und ein daran angeordnetes Wälzlager umfassen. Die Zughülse und das daran angeordnete Wälzlager können zum Einsetzen in die Aufnahme der Zugklaue des Lenkgetriebes ausgebildet sein. Die Zughülse kann im mit der Zugklaue gekoppelten Zustand die Bewegungen der Zugklaue entlang der Längsachse des chirurgischen Instruments auf eine Zugstange des chirurgischen Instruments übertragen.

Die Lenkeinrichtung kann wenigstens ein Zahnrad aufweisen, das an der Schaftwelle angeordnet und mit dem Stirnrad des Lenkgetriebes in Eingriff bringbar ist. Mit dem an der Schaftwelle angeordneten Zahnrad kann die Schaftwelle über das Stirnrad des Lenkgetriebes drehangetrieben werden, um die Schaftwelle in eine Rotation versetzen.

Die vorliegende Erfindung betrifft ferner ein steriles Antriebssystem für ein chirurgisches Instrument der voranstehend beschriebenen Art. Das Antriebssystem weist ein Lenkgetriebe und eine Antriebseinheit auf, die an einem Roboterarm anbringbar ist. Das Antriebssystem umfasst ferner wenigstens einen sterilen Überzug, der zumindest die Antriebseinheit umgibt, wobei der sterile Überzug eine Schnittstelle der Antriebseinheit freigibt. Das sterile Lenkgetriebe ist direkt mit der Schnittstelle an der Antriebseinheit koppelbar, wobei die Schnittstelle durch das Lenkgetriebe abgedeckt wird. Das sterile chirurgische Instrument kann mit der Schnittstelle an dem Lenkgetriebe austauschbar gekoppelt werden.

Wie voranstehend beschrieben wurde, weist das Lenkgetriebe neben der Schnittstelle für das chirurgische Instrument auch eine weitere Schnittstelle zur Kopplung mit der Schnittstelle der Antriebseinheit auf, die an einem Roboterarm anbringbar ist. Das Lenkgetriebe kann direkt mit der Schnittstelle der Antriebseinheit gekoppelt werden. Dies bedeutet, dass die Kopplung ohne zwischengeschaltete Elemente wie zum Beispiel einem Steriladapter erfolgen kann. Mit anderen Worten deckt das Lenkgetriebe die unsterile Schnittstelle an der Antriebseinheit unmittelbar ab. Das Lenkgetriebe kann während einer Operation durchgängig mit der unsterilen Schnittstelle der Antriebseinheit gekoppelt bleiben und diese abdecken.

Die Antriebseinheit kann beispielsweise über eine Führungseinrichtung an dem Roboterarm angebracht sein.

Der sterile Überzug kann beispielsweise von einer Folie gebildet werden. Der sterile Überzug kann ein Fenster aufweisen, das die Schnittstelle an der Antriebseinheit freigibt, sodass diese direkt mit dem Lenkgetriebe gekoppelt und von diesem abgedeckt werden kann. Durch diese Abdeckung der unsterilen Schnittstelle der Antriebseinheit mit dem Lenkgetriebe kann der sterile Überzug relativ einfach gestaltet sein. Es ist dementsprechend nicht nötig einen Steriladapter in den sterilen Überzug zu integrieren oder einen Steriladapter mit dem sterilen Überzug zu verbinden.

Zudem betrifft die vorliegende Erfindung ein Verfahren zur Montage eines sterilen Antriebssystems für ein voranstehend beschriebenes chirurgisches Instrument, wobei das Antriebssystem ein steriles Lenkgetriebe und eine an einem Roboterarm anbringbare Antriebseinheit aufweist. Das Verfahren umfasst die folgenden Schritte:
Anbringen eines sterilen Überzugs zumindest an der Antriebseinheit, wobei der sterile Überzug eine Schnittstelle der Antriebseinheit freigibt,
Anbringen des sterilen Lenkgetriebes an der Schnittstelle der Antriebseinheit, wobei das sterile Lenkgetriebe direkt mit der Schnittstelle gekoppelt wird, und wobei das sterile Lenkgetriebe die Schnittstelle der Antriebseinheit abdeckt, und
Koppeln eines sterilen chirurgischen Instruments mit der Schnittstelle an dem Lenkgetriebe, wobei das chirurgische Instrument austauschbar ist.

Das sterile Lenkgetriebe kann direkt und damit ohne zwischengeschaltete Elemente an der Schnittstelle der Antriebseinheit angebracht und mit dieser gekoppelt werden. Es ist kein Steriladapter für die Schnittstelle an der Antriebseinheit nötig, da diese Schnittstelle von dem Lenkgetriebe vollständig abgedeckt wird. Es liegt somit eine unmittelbare Kopplung zwischen dem Lenkgetriebe und der Schnittstelle der Antriebseinheit vor.

Im Folgenden wird die Erfindung anhand von Figuren beispielhaft erläutert. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es stellen dar:
- Figur 1: eine perspektivische Ansicht eines Lenkgetriebes gemäß einer Ausführungsform der Erfindung;
- Figur 2: eine weitere perspektivische Ansicht des Lenkgetriebes gemäß Figur 1;
- Figur 3: eine perspektivische Ansicht eines chirurgischen Instruments gemäß einer Ausführungsform der Erfindung;
- Figur 4: eine perspektivische Ansicht einer an einem Roboterarm anbringbaren Antriebseinheit mit einem sterilen Überzug;
- Figur 5: eine perspektivische Ansicht der Antriebseinheit gemäß Figur 4 im mit dem Lenkgetriebe gekoppelten Zustand;
- Figur 6: eine perspektivische Ansicht der Antriebseinheit, des Lenkgetriebes und des chirurgischen Instruments im gekoppelten Zustand; und
- Figur 7: eine teilweise aufgebrochene Ansicht der Antriebseinheit, des Lenkgetriebes und des chirurgischen Instruments im gekoppelten Zustand.

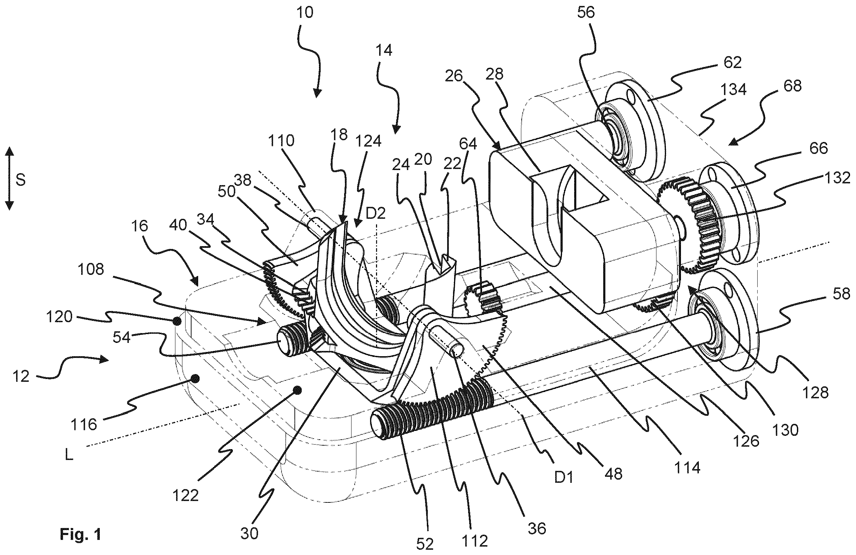

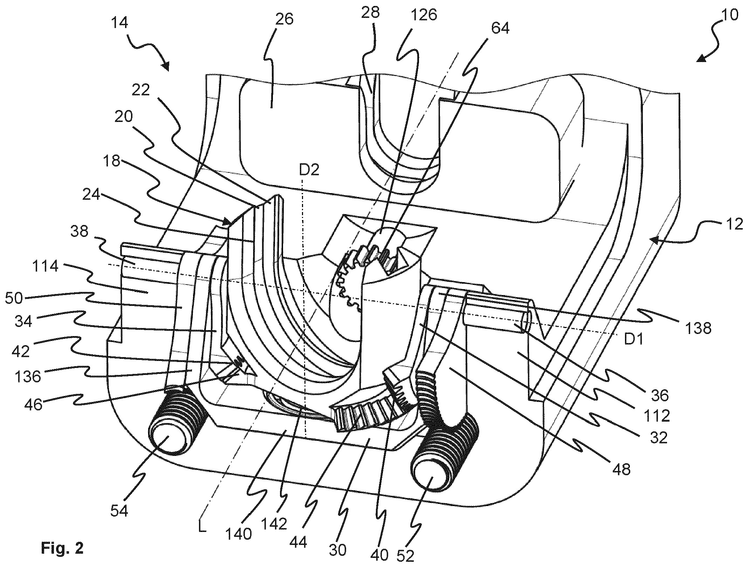

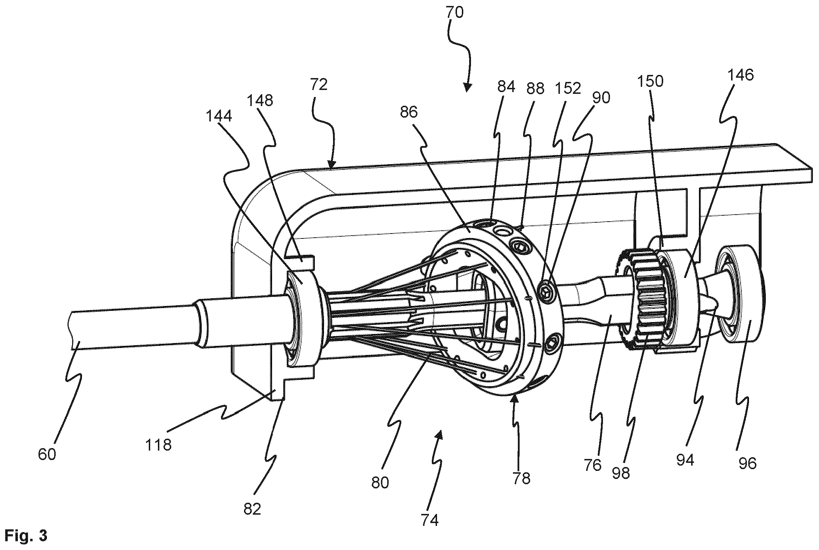

Die Figuren 1 und 2 zeigen perspektivische Ansichten eines Lenkgetriebes, das allgemein mit 10 bezeichnet ist. Das Lenkgetriebe 10 weist ein Getriebegehäuse 12 und eine Schnittstelle 14 auf, die zur Kopplung mit einem chirurgischen Instrument 70 (siehe Figur 3) ausgebildet ist.

Die Schnittstelle 14 umfasst einen Gehäusebereich 16 des Getriebegehäuses 12, der zur Kopplung mit einem Gehäuse 72 des chirurgischen Instruments 70 (siehe Figur 3) ausgebildet ist.

Der Gehäusebereich 16 weist einen Aufnahmebereich 108, zwei Lagerabschnitte 110 und 112 sowie eine Aussparung 114 auf. Die Aussparung 114 erstreckt sich an der Außenseite 116 des Gehäuses 12 entlang des Gehäusebereichs 16. Die Aussparung 114 ist derart ausgebildet, dass ein Rand 118 des Gehäuses 72 des chirurgischen Instruments 70 bündig mit der Außenseite 116 des Getriebegehäuses 12 abschließen kann. Die Aussparung 114 bildet eine Anlagefläche 120 für den Rand 118 des Gehäuses 72 aus (siehe Figur 3).

Der Aufnahmebereich 108 ist zwischen den beiden Lagerabschnitte 110 und 112 ausgebildet und erstreckt sich von einer Oberseite 122 des Gehäusebereichs 16 in das Getriebegehäuse 12 hinein. Der Aufnahmebereich 108 dient zur Aufnahme verschiedener Komponenten und Elemente des Lenkgetriebes 10, die nachstehend erläutert werden. Die Lagerabschnitte 110 und 112 stehen im wesentlichen in der Richtung S von der Oberseite 122 vor.

Die Schnittstelle 14 umfasst eine drehbar gelagerte Lenkgabel 18 zur Betätigung einer Lenkeinrichtung 74 des chirurgischen Instruments 70. Die Lenkgabel 18 ist in der Richtung S mit der Taumelscheibe 78 des chirurgischen Instruments 70 in Eingriff bringbar. Die Lenkgabel 18 ist in der Richtung S geöffnet. Anders ausgedrückt ist die Lenkgabel 18 nach oben geöffnet. Die Lenkgabel 18 weist dementsprechend eine Einführöffnung 124 auf, über die die Taumelscheibe 78 des chirurgischen Instruments 70 in Richtung S in die Lenkgabel 18 eingesetzt werden kann. Die Lenkgabel 18 weist eine Nut 20 auf, die axiale Kontaktflächen 22 und 24 für die Taumelscheibe 78 aufweist. Die Kontaktflächen 22 und 24 liegen einander gegenüber und erstrecken sich parallel zueinander. Die Nut 20 ist an der Innenseite der Lenkgabel 18 ausgebildet und erstreckt sich entlang der Innenseite der Lenkgabel 18.

Die Schnittstelle 14 weist ferner eine Zugklaue 26 auf. Die Zugklaue 26 kann mit der Lenkeinrichtung 74 des chirurgischen Instruments 70 gekoppelt werden. Die Zugklaue 26 hat eine Aufnahme 26, die in der Richtung S, d.h. nach oben geöffnet ist. Dadurch kann die Zugklaue 26 in Richtung S mit der Lenkeinrichtung 74 des chirurgischen Instruments 70 in Eingriff gebracht werden.

Das Lenkgetriebe 10 weist ein erstes Antriebsrad 32 (siehe Figur 2) und ein zweites Antriebsrad 34 auf. Dem ersten Antriebsrad 32 und dem zweiten Antriebsrad 34 ist jeweils eine Lagerwelle 36, 38 zugeordnet. Über die Lagerwellen 36 und 38 sind das erste Antriebsrad 32 und das zweite Antriebsrad 34 an jeweils einem der Lagerabschnitte 110 und 112 des Gehäusebereich 16 drehbar gelagert. Die Lagerwellen 36 und 38 legen eine erste Drehachse D1 fest.

Das Lenkgetriebe 10 weist einen Haltebügel 30 auf, der an den Lagerwellen 36 und 38 um die erste Drehachse D1 drehbar gelagert ist. Der Haltebügel 30 ist über die Lagerwellen 36 und 38 an den Lagerabschnitten 110 und 112 des Gehäusebereichs 16 drehbar gelagert. An dem Haltebügel 30 ist die Lenkgabel 18 um eine zweite Drehachse D2 drehbar gelagert. Bereits in Figur 1 ist erkennbar, dass sich die zweite Drehachse D2 und die erste Drehachse D1 schneiden. Beide Drehachsen D1 und D2 stehen senkrecht aufeinander.

Das Lenkgetriebe 10 weist ferner ein erstes Zahnrad 48 und ein zweites Zahnrad 50 auf. Das erste Zahnrad 48 und das zweite Zahnrad 50 sind an jeweils einer der Lagerwellen 36 und 38 drehbar gelagert. Die Zahnräder 48, 50 sind somit um die erste Drehachse D1 drehbar. Das erste Zahnrad 48 steht mit einer ersten Antriebsspindel 52 in Eingriff. Das zweite Zahnrad 50 steht mit einer zweiten Antriebsspindel 54 in Eingriff. Die Antriebsspindeln 52 und 54 treiben die Zahnräder 48 und 50 an, um die Lenkgabel 18 verlagern zu können. Die Zugklaue 26 ist mit einer dritten Antriebsspindel 56 verbunden, die die Zugklaue 26 entlang der Längsachse L verlagern kann.

Die Antriebsspindeln 52, 54 und 56 erstrecken sich parallel zur Längsachse des Lenkgetriebes 10 durch das Getriebegehäuse 12. An den Enden der Antriebsspindeln 52, 54 und 56 ist jeweils eine Antriebsscheibe 58, 62 angeordnet, die jeweils mit einer Motortreibscheibe (nicht gezeigt) koppelbar sind. In Figur 1 sind nur die Antriebsscheiben 58, 62 an den Antriebsspindeln 52 und 56 gezeigt. Die an der Antriebsspindel 54 angeordnete Antriebsscheibe ist in Figur 1 nicht gezeigt.

Das Lenkgetriebe 10 weist ein Stirnrad 64 zum Antreiben eines Schafts 60 des chirurgischen Instruments 70 über ein Zahnrad 98 an einer Schaftwelle 76 des chirurgischen Instruments 70 (siehe Figur 3) auf. Das Stirnrad 64 ist über die Welle 126 und ein Zahnradgetriebe 128, das die Zahnräder 130 und 132 umfasst, mit einer Antriebsscheibe 66 verbunden. Durch das Zahnradgetriebe 128 kann ein Versatz in der Richtung S zwischen dem Stirnrad 64 und der Antriebsscheibe 66 ausgeglichen werden.

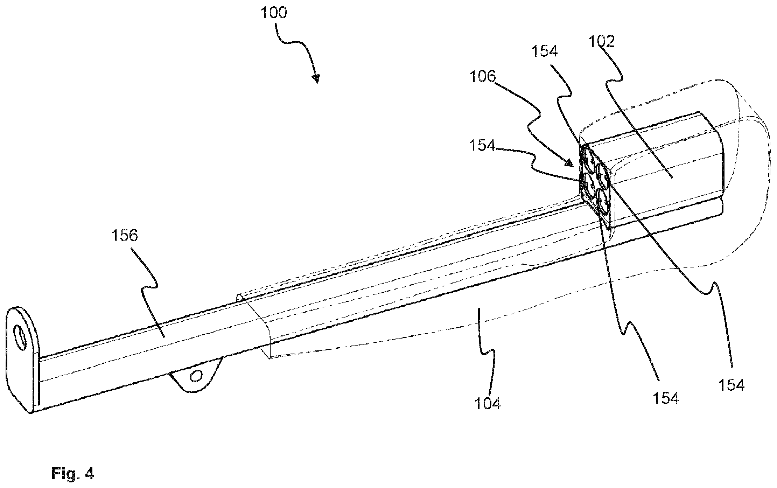

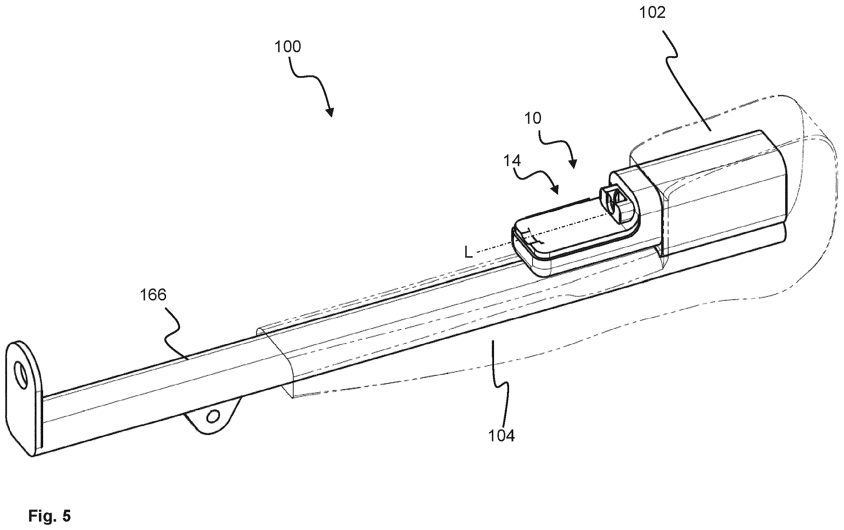

Das Lenkgetriebe 10 weist ferner eine weitere Schnittstelle 68 auf. Die Schnittstelle 68 ist zur Kopplung mit einer Schnittstelle 106 einer Antriebseinheit 102 ausgebildet sein, die an einem Roboterarm anbringbar ist (siehe Figur 4). Die Schnittstelle 68 umfasst die voranstehend beschriebenen Antriebsscheiben 58, 62, 66. Die Antriebsscheiben 58, 62 und 66 sind mit korrespondierenden Motortreibscheiben an der Schnittstelle 106 der Antriebseinheit 102 drehmomentübertragend koppelbar. Die Schnittstelle 68 an dem Lenkgetriebe 10 ist derart ausgebildet, dass die Schnittstelle 106 an der Antriebseinheit 102 von dem Lenkgetriebe 10 verdeckt werden kann (siehe Figur 5). Die Kopplung zwischen dem Lenkgetriebe 10 und der Antriebseinheit 102 erfolgt in Richtung der Längsachse L. Die Kopplung erfolgt demensprechend auch in Richtung der Drehachsen der Antriebsspindeln 52, 54, 56, da sich diese parallel zur Längsachse L erstrecken. Das Zahnradgetriebe 128 kann in Längsrichtung L zwischen der Zugklaue 26 und der Schnittstelle 68 mit den Antriebsscheiben 58, 62 und 66 angeordnet sein.

Das Lenkgetriebe 10 weist somit nicht nur die erste Schnittstelle 14 sondern auch eine zweite Schnittstelle 68 auf. Die zweite Schnittstelle 68 kann an einem in Richtung der Längsachse L des Lenkgetriebes 10 von der ersten Schnittstelle 14 abgewandten Ende des Getriebegehäuses 12 ausgebildet. Die Schnittstelle 68 kann an einer sich senkrecht zur Längsachse L erstreckenden Kopplungsfläche 134 des Getriebegehäuses 12 ausgebildet sein. Die Antriebsscheiben 58, 62 und 66 können quer und/oder in der Richtung S senkrecht zur Längsachse L versetzt zueinander an der Kopplungsfläche 134 angeordnet sein.

In Figur 2 ist verglichen mit Figur 1 deutlich ersichtlich, dass die Lenkgabel 18 Kegelverzahnungsabschnitte 44 und 46 aufweist. Gleiches gilt für die Kegelverzahnungen 40, 42 an den Antriebsrädern 32, 34, die in Figur 2 gezeigt sind.

In der Ansicht gemäß Figur 2 sind die Kegelverzahnungsabschnitte 44 und 46 einstückig mit der Lenkgabel 18 ausgebildet. Die Kegelverzahnungsabschnitte 44 und 46 können jedoch auch als von der Lenkgabel 18 unabhängiges Element oder als von der Lenkgabel 18 unabhängige Elemente ausgebildet sein. In diesem Fall können die Kegelverzahnungsabschnitte 44 und 46 zumindest eine Verbindungseinrichtung (nicht gezeigt) aufweisen, die zur drehmomentübertragenden Verbindung der Kegelverzahnungsabschnitte 44 und 46 mit der Lenkgabel 18 dienen können.

Die Kegelverzahnungsabschnitte 44, 46 an der Lenkgabel 18 stehen mit den Kegelverzahnungen 40, 42 an den Antriebsrädern 32, 34 in Eingriff. Aus den Figuren 1 und 2 wird ersichtlich, dass die Kegelverzahnungsabschnitte 44, 46 an der Lenkgabel 18 und die Kegelverzahnungen 40, 42 an den Antriebsrädern 32, 34 nur segmentweise ausgebildet sind. Durch die segmentweise Ausbildung der Kegelverzahnungsabschnitte 44, 46 und der Kegelverzahnungen 40, 42 kann Bauraum eingespart werden. Ferner können durch die segmentweise Ausbildung der Kegelverzahnungsabschnitte 44, 46 und der Kegelverzahnungen 40, 42 Kollisionen mit den Lenkdrähten 80 des chirurgischen Instruments 70 verhindert werden.

In Figur 2 ist die Anordnung der Antriebsräder 32, 34, der Zahnräder 48, 50 des Haltebügel 30 und der Lenkgabel 18 gezeigt. Entlang der Drehachse D1 einwärts sind zunächst die Zahnräder 48 und 50 angeordnet, die von den Antriebsspindeln 52 und 54 angetrieben werden. Entlang der Drehachse D1 einwärts ist der Haltebügel 30 an den Lagerwellen 36 und 38 angeordnet.

Der Haltebügel 30 weist zwei Verbindungsabschnitte 136 und 138 auf, über die der Haltebügel 30 drehbar an den Lagerwellen 36 und 38 angebracht ist. Die Verbindungsabschnitte 136 und 138 erstrecken sich im Wesentlichen parallel zueinander und parallel zur zweiten Drehachse D2. Die beiden Verbindungsabschnitte 136 und 138 werden über einen Lagerabschnitt 140 miteinander verbunden. Der Lagerabschnitt 140 bildet den Boden des Haltebügels 30. Der Lagerabschnitt 140 wird deshalb als Lagerabschnitt bezeichnet, da er zur drehbaren Lagerung der Lenkgabel 18 ausgebildet ist. Die Lenkgabel 18 ist um die zweite Drehachse D2 drehbar an dem Lagerabschnitt 140 des Haltebügels 30 gelagert. Der Haltebügel 30 weist dazu an seinem Lagerabschnitt 140 eine Lagervorrichtung 142 für die Lenkgabel 18 auf. Eine derartige Lagervorrichtung 142 kann beispielsweise ein Wälzlager sein. Die Drehachsen D1 und D2 schneiden sich senkrecht. Dadurch wird mittels des Haltebügels 30 eine kardanische Lagerung der Lenkgabel 18 gebildet, die in Richtung S nach oben geöffnet ist.

In Richtung der Drehachse D1 innerhalb des Haltebügels 30 bzw. zwischen den Verbindungsabschnitten 136 und 138 des Haltebügels 30 und der Lenkgabel 18 sind die Antriebsräder 32 und 34 mit ihren Kegelverzahnungen 40, 42 angeordnet. Die Kegelverzahnungen 40, 42 stehen mit den Kegelverzahnungsabschnitte 44, 46 an der Lenkgabel 18 in Eingriff.

Figur 3 zeigt eine perspektivische Darstellung des chirurgischen Instruments 70. Das chirurgische Instrument 70 weist ein Gehäuse 72 auf. In dem Gehäuse 72 ist eine Lenkeinrichtung 74 angeordnet. Die Lenkeinrichtung 74 weist eine Schaftwelle 76, die Taumelscheibe 78 und die mit der Taumelscheibe 78 verbundene Lenkdrähte 80 auf. An der Schaftwelle 76 sind eine Zughülse 94, ein Wälzlager 86 und ein Zahnrad 98 angeordnet, das dazu dient, den Schaft 60 in eine Rotation zu versetzen. Die Zughülse 94 und das daran angeordnete Wälzlager 94 können in die Aufnahme 28 der Zugklaue 26 des Lenkgetriebes 10 eingesetzt werden.

Die Lenkeinrichtung 74 ist über Wälzlager 144 und 146 drehbar an dem Gehäuse 72 gelagert. Dazu sind entsprechende Lagerstellen 148 und 150 an dem Gehäuse 72 ausgebildet. Das Gehäuse 72 hat ferner die Öffnung 82, über die die Lenkeinrichtung 74 mit dem Lenkgetriebe 10 koppelbar ist. Die Öffnung 82 wird zumindest teilweise durch den Rand 118 des Gehäuses 72 festgelegt. Der Rand 118 gelangt mit der Aussparung 114 an dem Getriebegehäuse 12 in Kontakt. Die Stirnfläche des Rands 118 kann sich dabei an die Anlagefläche 120 anlegen.

Die Taumelscheibe 78 weist einen umlaufenden Vorsprung 84 auf. Der umlaufende Vorsprung 84 hat axiale Kontaktflächen 86 und 88 für die Lenkgabel 18. Die Kontaktflächen 86 und 88 weisen voneinander weg und erstrecken sich parallel zueinander. Wenn die Taumelscheibe 78 mit der Lenkgabel 18 gekoppelt ist, wird der umlaufende Vorsprung 84 in der Nut 20 der Lenkgabel 18 aufgenommen. Die axialen Kontaktflächen 22, 24 der Nut 20 und die axialen Kontaktflächen 86 und 88 an dem umlaufenden Vorsprung 84 stehen in diesem Fall miteinander in Kontakt und können sich aneinander abstützen. Durch diesen axialen flächigen Kontakt zwischen den Kontaktflächen 22, 24, 86 und 88 wird bei einer Rotation und/oder Kippbewegung ein Verkanten der Taumelscheibe 78 im mit der Lenkgabel 18 gekoppelten Zustand verhindert.

Zudem weist der umlaufende Vorsprung 84 Befestigungselemente 90 zur Befestigung der Lenkdrähte 80 an der Taumelscheibe 78 auf. Dazu sind in dem umlaufenden Vorsprung 84 sich in radialer Richtung erstreckende Öffnungen 152 ausgebildet. In den Öffnungen 152 sind die Befestigungselemente 90 aufgenommen. Die Befestigungselemente 90 können Schrauben sein.

Figur 4 zeigt eine perspektivische Ansicht einer an einem Roboterarm (nicht gezeigt) anbringbaren Antriebseinheit 102. Die Antriebseinheit 102 weist eine Schnittstelle 106 zur Kopplung mit dem Lenkgetriebe 10 auf. An der Schnittstelle 106 sind vier Motortreibscheiben 154 angeordnet, die mit den Antriebsscheiben 58, 62 und 66 koppelbar sind, um die entsprechenden Antriebsspindeln 52, 54 und 56 des Lenkgetriebes 10 anzutreiben. Wie bereits erwähnt wurde, ist eine der Antriebsscheiben in den Figuren 1 und 2 nicht gezeigt. Das Lenkgetriebe 10 bzw. die zweite Schnittstelle 68 des Lenkgetriebes 10 umfasst jedoch vier Antriebsscheiben, die mit den Motortreibscheiben 154 gekoppelt werden können. Die Antriebseinheit 102 und auch Teile der daran angeordneten Führungseinrichtung 156, über die die Antriebseinheit 102 an dem Roboterarm anbringbar ist, sind gemäß Figur 4 mit einem sterilen Überzug 104 überzogen. Der sterile Überzug 104 umschließt die Antriebseinheit 102, gibt dabei jedoch die Schnittstelle 106 frei. Dadurch kann die zweite Schnittstelle 68 des Lenkgetriebes 10 direkt mit der Schnittstelle 106 der Antriebseinheit 102 gekoppelt werden. Dies bedeutet, dass die unsterilen Antriebe mit ihren unsterilen Motortreibscheiben 154 nicht von dem sterilen Überzug 104 oder einem zusätzlichen Steriladapter abgedeckt werden müssen, da die unsterile Schnittstelle 106 der Antriebseinheit 102 durch das Lenkgetriebe 10 abgedeckt wird.

Figur 5 zeigt eine perspektivische Ansicht der Antriebseinheit 102 im mit dem Lenkgetriebe 10 gekoppelten Zustand. Das Lenkgetriebe 10 ist steril. Das Lenkgetriebe 10 wird über seine zweite Schnittstelle 68 mit der Schnittstelle 106 der Antriebseinheit 102 gekoppelt. Diese Kopplung erfolgt in Richtung der Längsachse L des Lenkgetriebes 10, d.h. in Richtung der Drehachsen der Antriebsspindeln, die sich parallel zur Längsachse L erstrecken. Das sterile Lenkgetriebe 10 deckt somit die unsterilen Antriebe der Antriebseinheit 102 ab. Das Lenkgetriebe 10 kann während einer Operation durchgängig an der Antriebseinheit 102 verbleiben und die unsterilen Antriebe der Antriebseinheit 102 abdecken.

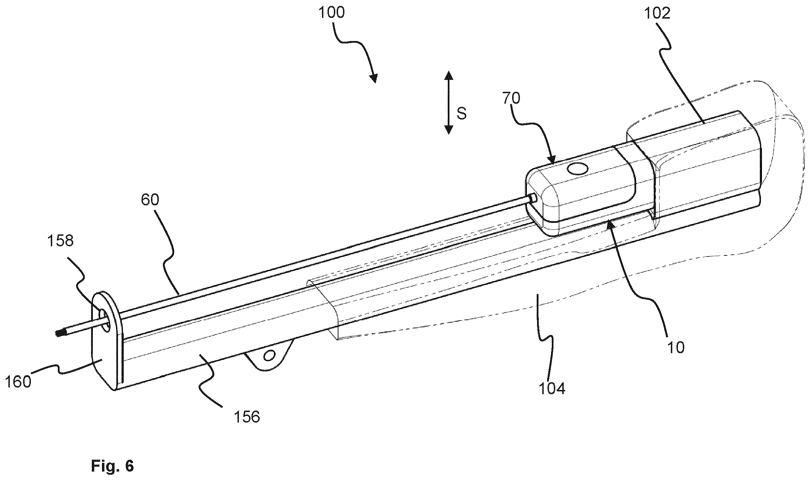

Figur 6 zeigt eine perspektivische Ansicht der Antriebseinheit 102, des damit gekoppelten Lenkgetriebes 10 und des nun mit dem Lenkgetriebe 10 gekoppelten chirurgischen Instrument 70. Das chirurgische Instrument 70 wurde in Richtung S auf das Lenkgetriebe 10 aufgesetzt und über die Schnittstelle 14 des Lenkgetriebes 10 (siehe Figuren 1 bis 5) mit dem Lenkgetriebe 10 gekoppelt. Der Schaft 60 des chirurgischen Instruments 70 erstreckt sich durch eine Führungsöffnung 158 an einem Führungsvorsprung 160 der Führungseinrichtung 156.

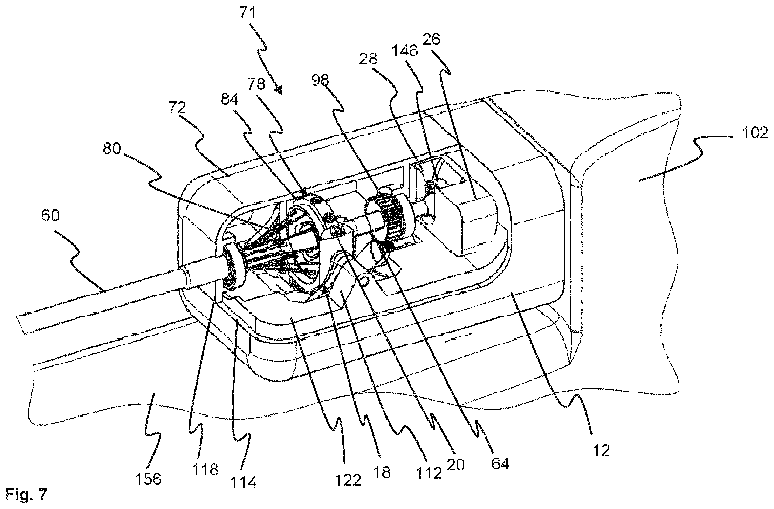

Figur 7 zeigt eine teilweise aufgebrochene perspektivische Ansicht, die das chirurgische Instrument 70, das Lenkgetriebe 10 und die Antriebseinheit 102 im miteinander gekoppelten Zustand zeigt. Das Lenkgetriebe 10 wurde mit seiner Schnittstelle 68 mit der Schnittstelle 106 der Antriebseinheit 102 gekoppelt. Das chirurgische Instrument 70 wurde in Richtung S auf das Lenkgetriebe 10 aufgesetzt. Dadurch gelangt der Gehäusebereich 16 des Gehäuses 12 des Lenkgetriebes 10 mit dem Gehäuse 72 des chirurgischen Instruments 70 in Eingriff. Der Rand 118 stützt sich dabei an der Aussparung 114 ab. Im mit dem Lenkgetriebe 10 gekoppelten Zustand des chirurgischen Instruments 70 ist die Taumelscheibe 78 in der Lenkgabel 18 und das Wälzlager 94 in der Aufnahme 28 der Zugklaue 26 aufgenommen. Der umlaufende Vorsprung 84 der Taumelscheibe 78 wird dabei in der Nut 20 der Lenkgabel 18 aufgenommen.

Das Stirnrad 64 des Lenkgetriebes 10 steht mit den Zahnrad 98 an der Schaftwelle 76 in Eingriff, um den Schaft 60 antreiben zu können. Im gekoppelten Zustand kann das chirurgische Instrument 70 über die Lenkgabel 18 gesteuert werden, deren Bewegungen über die Taumelscheibe 78 und die Lenkdrähte 80 auf das chirurgische Instrument bzw. das eigentliche Werkzeug (nicht gezeigt) übertragen werden.

Im gekoppelten Zustand des Lenkgetriebes 10 und des chirurgischen Instruments 70 können die Bewegungen der Lenkgabel 18 und Taumelscheibe 78 wie folgt durch das Lenkgetriebe 10 bewirkt werden:
Wenn die Antriebsspindeln 52 und 54 gegenläufig angetrieben werden, wodurch sich die Zahnräder 48 und 50 um die erste Drehachse D1 in entgegengesetzter Richtung bewegen, kann die Lenkgabel 18 um die zweite Drehachse D2 verdreht werden.

Werden die Antriebsspindeln 52 und 54 in gleicher Drehrichtung angetrieben, sodass sich die Zahnräder 48 und 50 in die gleiche Richtung bewegen, wird der Haltebügel 30 und damit die Lenkgabel 18 um die erste Drehachse D1 verschwenkt. Diese Schwenkbewegung resultiert daraus, dass bei einem Antreiben der Antriebsspindeln 52 und 54 in gleicher Drehrichtung die miteinander in Eingriff stehenden Kegelverzahnungsabschnitte 44, 46 und die Kegelverzahnungen 40, 42 keine Rotationsbewegung der Lenkgabel 18 um die zweite Drehachse D2 auslösen. Da sich die Drehachsen D1 und D2 schneiden, kann eine kardanische Lagerung der Lenkgabel 18 bereitgestellt werden.

Durch die beschriebenen Bewegungen der Lenkgabel 18 kann die Taumelscheibe 68 des chirurgischen Instruments 70 verlagert und dadurch räumlich verstellt werden. Die Bewegungen der Taumelscheibe 78 werden über die Lenkdrähte 80 auf die Schwenkglieder des eigentlichen Werkzeugs (nicht gezeigt) an dem chirurgischen Instrument 70 übertragen.

Die vorliegende Erfindung betrifft ein Lenkgetriebe 10 für ein chirurgisches Instrument 70, wobei das Lenkgetriebe 10 ein Getriebegehäuse 12 und eine Schnittstelle 14 zur Kopplung mit einem chirurgischen Instrument 70 aufweist, wobei die Schnittstelle 14 derart ausgebildet ist, dass das chirurgische Instrument 70 in einer Richtung S senkrecht zur Längsachse L des Lenkgetriebes 10 mit dem Lenkgetriebe 10 koppelbar ist, wobei die Schnittstelle 14 umfasst: einen Gehäusebereich 16 des Getriebegehäuses 12, der zur Kopplung mit einem Gehäuse 72 des chirurgischen Instruments 70 ausgebildet ist, und eine drehbar gelagerte Lenkgabel 18 zur Betätigung einer Lenkeinrichtung 74 des chirurgischen Instruments 70, wobei die drehbar gelagerte Lenkgabel 18 mit einer Taumelscheibe 78 der Lenkeinrichtung 74 des chirurgischen Instruments 70 über die Schnittstelle 14 koppelbar ist. Ferner betrifft die vorliegende Erfindung ein chirurgisches Instrument, ein steriles Antriebssystem und ein Verfahren zur Montage eines solchen Antriebssystems.

### Bezugszeichenliste

- 10: Lenkgetriebe
- 12: Getriebegehäuse
- 14: Schnittstelle
- 16: Gehäusebereich
- 18: Lenkgabel
- 20: Nut
- 22, 24: Kontaktflächen
- 26: Zugklaue
- 28: Aufnahme
- 30: Haltebügel
- 32: erstes Antriebsrad
- 34: zweites Antriebsrad
- 36, 38: Lagerwellen
- 40, 42: Kegelverzahnungen
- 44, 46: Kegelverzahnungsabschnitte
- 48: erstes Zahnrad
- 50: zweites Zahnrad
- 52, 54, 56: Antriebsspindeln
- 58: Antriebsscheibe
- 60: Schaft
- 62: Antriebsscheibe
- 64: Stirnrad
- 66: Antriebsscheibe
- 68: Schnittstelle
- 70: chirurgisches Instrument
- 72: Gehäuse
- 74: Lenkeinrichtung
- 76: Schaftwelle
- 78: Taumelscheibe
- 80: Lenkdrähte
- 82: Öffnung
- 84: Umlaufender Vorsprung
- 86, 88: Kontaktflächen
- 90: Befestigungselemente
- 92: Zughülse
- 94: Wälzlager
- 98: Zahnrad
- 100: Antriebssystem
- 102: Antriebseinheit
- 104: Steriler Überzug
- 106: Schnittstelle
- 108: Aufnahmebereich
- 110, 112: Lagerabschnitte
- 114: Aussparung
- 116: Außenseite
- 118: Rand
- 120: Anlagefläche
- 122: Oberseite
- 124: Einführöffnung
- 126: Welle
- 128: Zahnradgetriebe
- 130, 132: Zahnräder
- 134: Kontaktfläche
- 136, 138: Verbindungsabschnitte
- 140: Lagerabschnitt
- 142: Lagervorrichtung
- 144, 146: Wälzlager
- 148, 150: Lagerstellen
- 152: Öffnungen
- 154: Motortreibscheiben
- 156: Führungseinrichtung
- 158: Führungsöffnung
- 160: Führungsvorsprung
- S: Richtung
- L: Längsachse
- D1: erste Drehachse
- D2: zweite Drehachse

## Patentansprüche

1. Lenkgetriebe (10) für ein chirurgisches Instrument (70),
wobei das Lenkgetriebe (10) ein Getriebegehäuse (12) und eine Schnittstelle (14) zur Kopplung mit einem chirurgischen Instrument (70) aufweist,
wobei die Schnittstelle (14) derart ausgebildet ist, dass das chirurgische Instrument (70) in einer Richtung (S) senkrecht zur Längsachse (L) des Lenkgetriebes (10) mit dem Lenkgetriebe (10) koppelbar ist,
wobei die Schnittstelle (14) umfasst:
einen Gehäusebereich (16) des Getriebegehäuses (12), der zur Kopplung mit einem Gehäuse (72) des chirurgischen Instruments (70) ausgebildet ist, und
eine drehbar gelagerte Lenkgabel (18) zur Betätigung einer Lenkeinrichtung (74) des chirurgischen Instruments (70), wobei die drehbar gelagerte Lenkgabel (18) mit einer Taumelscheibe (78) der Lenkeinrichtung (74) des chirurgischen Instruments (70) über die Schnittstelle (14) koppelbar ist.

2. Lenkgetriebe (10) nach Anspruch 1,
wobei die Lenkgabel (18) derart ausgebildet ist, dass die Lenkgabel (18) in einer Richtung (S) senkrecht zur Längsachse (L) des Lenkgetriebes (10) mit der Taumelscheibe (78) des chirurgischen Instruments (70) in Eingriff bringbar ist.

3. Lenkgetriebe nach Anspruch 1 oder 2,
wobei die Lenkgabel (18) eine Nut (20) hat, die axiale Kontaktflächen (22, 24) für die Taumelscheibe (78) aufweist.

4. Lenkgetriebe (10) nach einem der Ansprüche 1 bis 3,
wobei die Schnittstelle (14) eine Zugklaue (26) aufweist, die zur Kopplung mit der Lenkeinrichtung (74) des chirurgischen Instruments (70) ausgebildet ist, wobei die Zugklaue (26) wenigstens eine Aufnahme (28) aufweist, die derart ausgebildet ist, dass die Zugklaue (26) in einer Richtung (S) senkrecht zur Längsachse (L) des Lenkgetriebes (10) mit der Lenkeinrichtung (74) des chirurgischen Instruments (70) in Eingriff bringbar ist.

5. Lenkgetriebe (10) nach einem der Ansprüche 1 bis 4,
wobei das Lenkgetriebe (10) wenigstens einen Haltebügel (30) aufweist, der um eine erste Drehachse (D1) drehbar an dem Getriebegehäuse (12) gelagert ist, wobei die Lenkgabel (18) um eine zweite Drehachse (D2) drehbar an dem Haltebügel (30) gelagert ist.

6. Lenkgetriebe (10) nach einem der Ansprüche 1 bis 5,
wobei das Lenkgetriebe (10) ein erstes Antriebsrad (32) und ein zweites Antriebsrad (34) aufweist, die jeweils über eine Lagerwelle (36, 38) an dem Getriebegehäuse (16) um die erste Drehachse (D1) drehbar gelagert sind, wobei das erste Antriebsrad (32) und das zweite Antriebsrad (34) jeweils eine Kegelverzahnung (40, 42) aufweisen.

7. Lenkgetriebe (10) nach Anspruch 6,
wobei die Lenkgabel (18) wenigstens zwei Kegelverzahnungsabschnitte (44, 46) aufweist, wobei die Kegelverzahnungsabschnitte (44, 46) der Lenkgabel (18) mit den Kegelverzahnungen (40, 42) an den Antriebsrädern (32, 34) in Eingriff stehen.

8. Lenkgetriebe nach Anspruch 6 oder 7,
wobei der Haltebügel (30) drehbar an den Lagerwellen (36, 38) der Antriebsräder (32, 34) gelagert ist.

9. Lenkgetriebe (10) nach einem der Ansprüche 6 bis 8,
wobei die zweite Drehachse (D2) der Lenkgabel (18) die von den Lagerwellen (36, 38) definierte erste Drehachse (D1) schneidet.

10. Lenkgetriebe (10) nach einem der Ansprüche 6 bis 9,
wobei das Lenkgetriebe (10) ein erstes Zahnrad (48), ein zweites Zahnrad (50), eine erste Antriebsspindel (52) und eine zweite Antriebsspindel (54) aufweist, wobei das erste Zahnrad (48) der ersten Antriebsspindel (52) und das zweite Zahnrad (50) der zweiten Antriebsspindel (54) zugeordnet ist, wobei das erste Zahnrad (48) und das zweite Zahnrad (50) an den Lagerwellen (36, 38) der Antriebsräder (32, 34) drehbar gelagert sind.

11. Lenkgetriebe nach einem der Ansprüche 4 bis 10,
wobei die Zugklaue (26) über eine dritte Antriebsspindel (56) antreibbar ist.

12. Lenkgetriebe nach Anspruch 10 oder 11,
wobei sich die Antriebsspindeln (52, 54, 56) parallel zur Längsachse (L) des Lenkgetriebes (10) durch das Getriebegehäuse (12) erstrecken und an ihren Enden jeweils eine Antriebsscheibe (58, 60, 62) aufweisen, die mit Motortreibscheiben koppelbar sind.

13. Lenkgetriebe (10) nach einem der Ansprüche 1 bis 12,
wobei die Schnittstelle (14) wenigstens ein Stirnrad (64) zum Antreiben eines Schafts (60) des chirurgischen Instruments (70) aufweist, wobei das Stirnrad (64) von einer weiteren Antriebsscheibe (66) antreibbar ist.

14. Lenkgetriebe (10) nach Anspruch 12 oder 13,
wobei das Lenkgetriebe (10) eine weitere Schnittstelle (68) zur Verbindung mit einer Antriebseinheit (102) aufweist, wobei diese Schnittstelle (68) die Antriebsscheiben (58, 60, 62, 66) umfasst.

15. Chirurgisches Instrument (70) zur Kopplung mit einer Schnittstelle (14) eines Lenkgetriebes (10) nach einem der Ansprüche 1 bis 14,
wobei das chirurgische Instrument (70) ein Gehäuse (72) und eine in dem Gehäuse (72) angeordnete Lenkeinrichtung (74) aufweist, wobei die Lenkeinrichtung (74) zumindest eine Schaftwelle (76), eine Taumelscheibe (78) und mit der Taumelscheibe (78) verbundene Lenkdrähte (80) aufweist, wobei das Gehäuse (72) eine Öffnung (82) aufweist, über die die Lenkeinrichtung (74) mit dem Lenkgetriebe (10) koppelbar ist.

16. Chirurgisches Instrument (70) nach Anspruch 15,
wobei die Taumelscheibe (78) einen umlaufenden Vorsprung (84) aufweist, der axiale Kontaktflächen (86, 88) für die Lenkgabel (18) aufweist.

17. Chirurgisches Instrument (70) nach Anspruch 16,
wobei der umlaufende Vorsprung (84) Befestigungselemente (90) zur Befestigung der Lenkdrähte (80) an der Taumelscheibe (78) aufweist.

18. Chirurgisches Instrument (70) nach einem der Ansprüche 15 bis 17,
wobei die Lenkeinrichtung (74) eine Zughülse (92) und ein daran angeordnetes Wälzlager (94) umfasst, wobei die Zughülse (92) und das daran angeordnete Wälzlager (94) zum Einsetzen in die Aufnahme (28) der Zugklaue (26) des Lenkgetriebes (10) ausgebildet sind.

19. Chirurgisches Instrument (70) nach einem der Ansprüche 15 bis 18,
wobei die Lenkeinrichtung (74) ein Zahnrad (98) aufweist, das an der Schaftwelle (76) angeordnet und mit dem Stirnrad (64) des Lenkgetriebes (10) in Eingriff bringbar ist.

20. Steriles Antriebssystem (100) für ein chirurgisches Instrument (70) nach einem der Ansprüche 15 bis 19, wobei das Antriebssystem (100) ein Lenkgetriebe (10) nach einem der Ansprüche 1 bis 14 und eine an einem Roboterarm anbringbare Antriebseinheit (102) aufweist, wobei das Antriebssystem (102) umfasst:
wenigstens einen sterilen Überzug (104), der zumindest die Antriebseinheit (102) umgibt, wobei der sterile Überzug (104) eine Schnittstelle (106) der Antriebseinheit (102) freigibt,
wobei das sterile Lenkgetriebe (10) mit der Schnittstelle (106) an der Antriebseinheit (102) direkt koppelbar ist, wobei die Schnittstelle (106) durch das Lenkgetriebe (10) abdeckt ist, und
wobei das sterile chirurgische Instrument (70) mit der Schnittstelle (14) an dem Lenkgetriebe (10) austauschbar gekoppelt ist.

21. System (100) nach Anspruch 20,
wobei der Überzug (104) eine Folie ist.

22. Verfahren zur Montage eines sterilen Antriebssystems (100) für ein chirurgisches Instrument (70) nach einem Ansprüche 15 bis 19, wobei das Antriebssystem (100) ein steriles Lenkgetriebe (10) nach einem der Ansprüche 1 bis 14 und eine an einem Roboterarm anbringbare Antriebseinheit (102) aufweist, wobei das Verfahren die folgenden Schritte umfasst:
Anbringen eines sterilen Überzugs (104) zumindest an der Antriebseinheit (102),
wobei der sterile Überzug (104) eine Schnittstelle (106) der Antriebseinheit (102) freigibt,
Anbringen des sterilen Lenkgetriebes (10) an der Schnittstelle (106) an der Antriebseinheit (102), wobei das sterile Lenkgetriebe (10) direkt mit der Schnittstelle (106) gekoppelt wird, und wobei das sterile Lenkgetriebe (10) die Schnittstelle (106) der Antriebseinheit (102) abdeckt, und
Koppeln eines sterilen chirurgischen Instruments (70) mit der Schnittstelle (14) an dem Lenkgetriebe (10), wobei das chirurgische Instrument (70) austauschbar ist.

## Claims

1. A steering mechanism (10) for a surgical instrument (70),
wherein the steering mechanism (10) has a mechanism housing (12) and an interface (14) for coupling to a surgical instrument (70),
wherein the interface (14) is designed in such manner that the surgical instrument (70) can be coupled to the steering mechanism (10) in a direction (S) perpendicular to the longitudinal axis (L) of the steering mechanism (10),
wherein the interface (14) comprises:
a housing region (16) of the mechanism housing (12), which is designed for coupling to a housing (72) of the surgical instrument (70), and a rotatably mounted steering fork (18) for actuating a steering device (74) of the surgical instrument (70), wherein the rotatably mounted steering fork (18) can be coupled to a swash plate (78) of the steering device (74) of the surgical instrument (70) via the interface (14).

2. The steering mechanism (10) according to claim 1,
wherein the steering fork (18) is designed in such manner that the steering fork (18) can be brought into engagement with the swash plate (78) of the surgical instrument (70) in a direction (S) perpendicular to the longitudinal axis (L) of the steering mechanism (10).

3. The steering mechanism according to claim 1 or 2,
wherein the steering fork (18) has a groove (20) which has axial contact surfaces (22, 24) for the swash plate (78).

4. The steering mechanism (10) according to one of claims 1 to 3,
wherein the interface (14) has a pull claw (26) which is designed for coupling with the steering device (74) of the surgical instrument (70), wherein the pull claw (26) has at least one receptacle (28) which is designed in such manner that the pull claw (26) can be brought into engagement with the steering device (74) of the surgical instrument (70) in a direction (S) perpendicular to the longitudinal axis (L) of the steering mechanism (10).

5. The steering mechanism (10) according to one of claims 1 to 4,
wherein the steering mechanism (10) has at least one retaining bracket (30) which is mounted on the mechanism housing (12) so as to be rotatable about a first axis of rotation (D1), wherein the steering fork (18) is mounted on the retaining bracket (30) so as to be rotatable about a second axis of rotation (D2).

6. The steering mechanism (10) according to one of claims 1 to 5,
wherein the steering mechanism (10) has a first drive gear (32) and a second drive gear (34), each of which is mounted on the mechanism housing (16) via a bearing shaft (36, 38) so as to be rotatable about the first axis of rotation (D1), wherein the first drive gear (32) and the second drive gear (34) each have a bevel toothing (40, 42).

7. The steering mechanism (10) according to claim 6,
wherein the steering fork (18) has at least two bevel toothing sections (44, 46), wherein the bevel toothing sections (44, 46) of the steering fork (18) are in engagement with the bevel toothings (40, 42) on the drive gears (32, 34).

8. The steering mechanism according to claim 6 or 7,
wherein the retaining bracket (30) is rotatably mounted on the bearing shafts (36, 38) of the drive gears (32, 34).

9. The steering mechanism (10) according to one of claims 6 to 8,
wherein the second axis of rotation (D2) of the steering fork (18) intersects the first axis of rotation (D1) defined by the bearing shafts (36, 38).

10. The steering mechanism (10) according to one of claims 6 to 9,
wherein the steering mechanism (10) has a first gear (48), a second gear (50), a first drive spindle (52) and a second drive spindle (54), wherein the first gear (48) is assigned to the first drive spindle (52) and the second gear (50) is assigned to the second drive spindle (54), wherein the first gear (48) and the second gear (50) are rotatably mounted on the bearing shafts (36, 38) of the drive gears (32, 34).

11. The steering mechanism according to one of claims 4 to 10,
wherein the pull claw (26) can be driven via a third drive spindle (56).

12. The steering mechanism according to claim 10 or 11,
wherein the drive spindles (52, 54, 56) extend through the mechanism housing (12) parallel to the longitudinal axis (L) of the steering mechanism (10) and each have, at their ends, a drive pulley (58, 60, 62), which can be coupled to motor drive pulleys.

13. The steering mechanism (10) according to one of claims 1 to 12,
wherein the interface (14) has at least one spur gear (64) for driving a shaft (60) of the surgical instrument (70), wherein the spur gear (64) can be driven by a further drive pulley (66).

14. The steering mechanism (10) according to claim 12 or 13,
wherein the steering mechanism (10) has a further interface (68) for connection to a drive unit (102), wherein this interface (68) comprises the drive pulleys (58, 60, 62, 66).

15. A surgical instrument (70) for coupling to an interface (14) of a steering mechanism (10) according to one of claims 1 to 14,
wherein the surgical instrument (70) has a housing (72) and a steering device (74) arranged in the housing (72), wherein the steering device (74) has at least one steering shaft (76), a swash plate (78) and steering wires (80) connected to the swash plate (78), wherein the housing (72) has an opening (82) via which the steering device (74) can be coupled to the steering mechanism (10).

16. The surgical instrument (70) according to claim 15,
wherein the swash plate (78) has a circumferential protrusion (84) which has axial contact surfaces (86, 88) for the steering fork (18).

17. The surgical instrument (70) according to claim 16,
wherein the circumferential protrusion (84) has fastening elements (90) for fastening the steering wires (80) to the swash plate (78).

18. The surgical instrument (70) according to one of claims 15 to 17,
wherein the steering device (74) comprises a pull sleeve (92) and a rolling bearing (94) arranged thereon, wherein the pull sleeve (92) and the rolling bearing (94) arranged thereon are designed for insertion into the receptacle (28) of the pull claw (26) of the steering mechanism (10).

19. The surgical instrument (70) according to one of claims 15 to 18,
wherein the steering device (74) has a gear (98) which is arranged on the steering shaft (76) and can be brought into engagement with the spur gear (64) of the steering mechanism (10).

20. A sterile drive system (100) for a surgical instrument (70) according to one of claims 15 to 19, wherein the drive system (100) has a steering mechanism (10) according to one of claims 1 to 14 and a drive unit (102) which can be attached to a robotic arm, wherein the drive system (102) comprises:
at least one sterile cover (104) which surrounds at least the drive unit (102), wherein the sterile cover (104) exposes an interface (106) of the drive unit (102),
wherein the sterile steering mechanism (10) can be directly coupled to the interface (106) on the drive unit (102), wherein the interface (106) is covered by the steering mechanism (10), and
wherein the sterile surgical instrument (70) is interchangeably coupled to the interface (14) on the steering mechanism (10).

21. A system (100) according to claim 20,
wherein the cover (104) is a film.

22. A method for assembling a sterile drive system (100) for a surgical instrument (70) according to one of claims 15 to 19, wherein the drive system (100) comprises a sterile steering mechanism (10) according to one of claims 1 to 14 and a drive unit (102) which can be attached to a robotic arm, wherein the method comprises the following steps:
Attaching a sterile cover (104) at least to the drive unit (102), wherein the sterile cover (104) exposes an interface (106) of the drive unit (102),
Attaching the sterile steering mechanism (10) to the interface (106) on the drive unit (102), wherein the sterile steering mechanism (10) is coupled directly to the interface (106), and wherein the sterile steering mechanism (10) covers the interface (106) of the drive unit (102), and
Coupling a sterile surgical instrument (70) to the interface (14) on the steering mechanism (10), wherein the surgical instrument (70) is interchangeable.

## Revendications

1. Mécanisme de direction (10) pour un instrument chirurgical (70),
dans lequel le mécanisme de direction (10) présente un boîtier de mécanisme (12) et une interface (14) pour l'accouplement avec un instrument chirurgical (70),
dans lequel l'interface (14) est conçue de telle sorte que l'instrument chirurgical (70) peut être accouplé au mécanisme de direction (10) dans une direction (S) perpendiculaire à l'axe longitudinal (L) du mécanisme de direction (10),
dans lequel l'interface (14) comprend :
une zone de boîtier (16) du boîtier de mécanisme (12), qui est conçue pour être accouplée à un boîtier (72) de l'instrument chirurgical (70), et une fourche de direction (18) montée rotative pour actionner un dispositif de direction (74) de l'instrument chirurgical (70), dans lequel la fourche de direction (18) montée rotative peut être accouplée à un disque oscillant (78) du dispositif de direction (74) de l'instrument chirurgical (70) par l'intermédiaire de l'interface (14).

2. Mécanisme de direction (10) selon la revendication 1,
dans lequel la fourche de direction (18) est conçue de telle sorte que la fourche de direction (18) peut être mise en prise avec le disque oscillant (78) de l'instrument chirurgical (70) dans une direction (S) perpendiculaire à l'axe longitudinal (L) du mécanisme de direction (10).

3. Mécanisme de direction selon la revendication 1 ou 2,
dans lequel la fourche de direction (18) a une rainure (20) qui présente des surfaces de contact axiales (22, 24) pour le disque oscillant (78).

4. Mécanisme de direction (10) selon l'une des revendications 1 à 3,
dans lequel l'interface (14) présente une griffe de traction (26) qui est conçue pour être accouplée au dispositif de direction (74) de l'instrument chirurgical (70), dans lequel la griffe de traction (26) présente au moins un logement (28) qui est conçu de telle sorte que la griffe de traction (26) peut être mise en prise avec le dispositif de direction (74) de l'instrument chirurgical (70) dans une direction (S) perpendiculaire à l'axe longitudinal (L) du mécanisme de direction (10).

5. Mécanisme de direction (10) selon l'une des revendications 1 à 4,
dans lequel le mécanisme de direction (10) présente au moins un étrier de maintien (30) qui est monté sur le boîtier de mécanisme (12) rotatif autour d'un premier axe de rotation (D1), dans lequel la fourche de direction (18) est montée sur l'étrier de maintien (30) rotative autour d'un second axe de rotation (D2).

6. Mécanisme de direction (10) selon l'une des revendications 1 à 5,
dans lequel le mécanisme de direction (10) présente une première roue motrice (32) et une seconde roue motrice (34), qui sont respectivement montées rotatives sur le boîtier de mécanisme (16) autour du premier axe de rotation (D1) par l'intermédiaire d'un arbre de palier (36, 38), dans lequel la première roue motrice (32) et la seconde roue motrice (34) présentent respectivement une denture conique (40, 42).

7. Mécanisme de direction (10) selon la revendication 6,
dans lequel la fourche de direction (18) présente au moins deux sections à denture conique (44, 46), dans lequel les sections à denture conique (44, 46) de la fourche de direction (18) sont en prise avec les dentures coniques (40, 42) des roues motrices (32, 34).

8. Mécanisme de direction selon la revendication 6 ou 7,
dans lequel l'étrier de maintien (30) est monté rotatif sur les arbres de palier (36, 38) des roues motrices (32, 34).

9. Mécanisme de direction (10) selon l'une des revendications 6 à 8,
dans lequel le second axe de rotation (D2) de la fourche de direction (18) coupe le premier axe de rotation (D1) défini par les arbres de palier (36, 38).

10. Mécanisme de direction (10) selon l'une des revendications 6 à 9,
dans lequel le mécanisme de direction (10) présente une première roue dentée (48), une seconde roue dentée (50), une première broche d'entraînement (52) et une deuxième broche d'entraînement (54), dans lequel la première roue dentée (48) est associée à la première broche d'entraînement (52) et la seconde roue dentée (50) est associée à la deuxième broche d'entraînement (54), dans lequel la première roue dentée (48) et la seconde roue dentée (50) sont montées rotatives sur les arbres de palier (36, 38) des roues motrices (32, 34).

11. Mécanisme de direction selon l'une des revendications 4 à 10,
dans lequel la griffe de traction (26) peut être entraînée par l'intermédiaire d'une troisième broche d'entraînement (56).

12. Mécanisme de direction selon la revendication 10 ou 11,
dans lequel les broches d'entraînement (52, 54, 56) s'étendent parallèlement à l'axe longitudinal (L) du mécanisme de direction (10) à travers le boîtier de mécanisme (12) et présentent respectivement à leurs extrémités un disque d'entraînement (58, 60, 62) qui peut être accouplé à des disques d'entraînement de moteur.

13. Mécanisme de direction (10) selon l'une des revendications 1 à 12,
dans lequel l'interface (14) présente au moins une roue cylindrique (64) pour entraîner une tige (60) de l'instrument chirurgical (70), dans lequel la roue cylindrique (64) peut être entraînée par un autre disque d'entraînement (66).

14. Mécanisme de direction (10) selon la revendication 12 ou 13,
dans lequel le mécanisme de direction (10) présente une autre interface (68) destinée à être reliée à une unité d'entraînement (102), dans lequel cette interface (68) comprend les disques d'entraînement (58, 60, 62, 66).

15. Instrument chirurgical (70) destiné à être accouplé à une interface (14) d'un mécanisme de direction (10) selon l'une des revendications 1 à 14,
dans lequel l'instrument chirurgical (70) présente un boîtier (72) et un dispositif de direction (74) agencé dans le boîtier (72), dans lequel le dispositif de direction (74) présente au moins un arbre de tige (76), un disque oscillant (78) et des fils de direction (80) reliés au disque oscillant (78), dans lequel le boîtier (72) présente une ouverture (82) par l'intermédiaire de laquelle le dispositif de direction (74) peut être accouplé au mécanisme de direction (10).

16. Instrument chirurgical (70) selon la revendication 15,
dans lequel le disque oscillant (78) présente une saillie périphérique (84) qui présente des surfaces de contact axiales (86, 88) pour la fourche de direction (18).

17. Instrument chirurgical (70) selon la revendication 16,
dans lequel la saillie périphérique (84) présente des éléments de fixation (90) pour la fixation des fils de direction (80) au disque oscillant (78).

18. Instrument chirurgical (70) selon l'une des revendications 15 à 17,
dans lequel le dispositif de direction (74) comprend un manchon de traction (92) et un palier à roulement (94) agencé sur celui-ci, dans lequel le manchon de traction (92) et le palier à roulement (94) agencé sur celui-ci sont conçus pour être insérés dans le logement (28) de la griffe de traction (26) du mécanisme de direction (10).

19. Instrument chirurgical (70) selon l'une des revendications 15 à 18,
dans lequel le dispositif de direction (74) présente une roue dentée (98) qui est agencée sur l'arbre de tige (76) et qui peut être mise en prise avec la roue cylindrique (64) du mécanisme de direction (10).

20. Système (100) d'entraînement stérile pour un instrument chirurgical (70) selon l'une des revendications 15 à 19, dans lequel le système (100) d'entraînement présente un mécanisme de direction (10) selon l'une des revendications 1 à 14 et une unité d'entraînement (102) pouvant être appliquée sur un bras robotisé, dans lequel le système d'entraînement (102) comprend :
au moins un revêtement stérile (104) qui entoure au moins l'unité d'entraînement (102), dans lequel le revêtement stérile (104) laisse libre une interface (106) de l'unité d'entraînement (102),
dans lequel le mécanisme de direction (10) stérile peut être accouplé directement à l'interface (106) sur l'unité d'entraînement (102), dans lequel l'interface (106) est recouverte par le mécanisme de direction (10), et
dans lequel l'instrument chirurgical (70) stérile est accouplé de manière interchangeable au mécanisme de direction (10) par l'interface (14).

21. Système (100) selon la revendication 20,
dans lequel le revêtement (104) est un film.

22. Procédé d'assemblage d'un système (100) d'entraînement stérile pour un instrument chirurgical (70) selon l'une des revendications 15 à 19, dans lequel le système (100) d'entraînement présente un mécanisme de direction (10) stérile selon l'une des revendications 1 à 14 et une unité d'entraînement (102) pouvant être appliquée sur un bras robotisé, dans lequel le procédé comprend les étapes suivantes :
application d'un revêtement (104) stérile au moins sur l'unité d'entraînement (102), dans lequel le revêtement stérile (104) laisse libre une interface (106) de l'unité d'entraînement (102),
application du mécanisme de direction (10) stérile sur l'interface (106) de l'unité d'entraînement (102), dans lequel le mécanisme de direction (10) stérile est accouplé directement à l'interface (106), et dans lequel le mécanisme de direction (10) stérile recouvre l'interface (106) de l'unité d'entraînement (102), et
accouplement d'un instrument chirurgical (70) stérile à l'interface (14) du mécanisme de direction (10), dans lequel l'instrument chirurgical (70) est interchangeable.
